# FASCICULE DE BREVET EUROPEEN

(11) **EP 0 607 088 B1**
(45) Date de publication et mention de la délivrance du brevet: **09.04.1997**
(21) Numéro de dépôt: 94400082.7
(22) Date de dépôt: 13.01.1994
(51) Int. Cl.: C07J 41/00, C07J 21/00, C07J 5/00

(54) **Nouveaux dérivés 17,20-époxydes du pregnane, leur application à la préparation de dérivés cortisoniques et intermédiaires**
Neue 17,20-Epoxyd Derivate von Pregnan, ihre Verwendung zur Herstellung von Cortison-Derivaten und Zwischenprodukte dafür
New 17,20-epoxy pregnane derivatives, their use in the preparation of cortisone derivatives and intermediates therefor

(30) Priorité: 14.01.1993 FR 9300290
(43) Date de publication de la demande: 20.07.1994
(73) Titulaire: ROUSSEL UCLAF, 93230 Romainville (FR)
(72) Inventeur: Buendia, Jean, F-94170 Le Perreux sur Marne (FR); Chauvin, Rémi, F-78230 Le Pecq (FR); Vivat, Michel, F-77400 Lagny sur Marne (FR)
(74) Mandataire: Vieillefosse, Jean-Claude

(56) Documents cités:
- EP-A- 0 515 264
- EP-A- 0 573 362
- DE-A- 2 330 581
- FR-A- 2 236 507
- US-A- 2 541 439
- US-A- 2 744 108
- US-A- 2 813 860
- US-A- 2 933 515
- US-A- 3 074 979
- US-A- 3 419 582
- US-A- 4 600 538
- POLISH JOURNAL OF CHEMISTRY vol. 56, no. 4-6 , 1982 , WARSAW, PL pages 741 - 747 M. GUMULKA ET AL 'Facile Synthesis of Corticosteroids from 17-keto-Steroids via 20-cyano-21-carboxylates'
- JOURNAL OF ORGANIC CHEMISTRY. vol. 55, no. 21 , 12 Octobre 1990 , EASTON US pages 5625 - 5631 HIDEO NEMOTO ET AL 'First Enantioselective Total Synthesis of (+)-Cortisone'
- TETRAHEDRON LETTERS. vol. 33, no. 36 , 1 Septembre 1992 , OXFORD GB pages 5265 - 5266 A. TOR ET AL 'Synthesis of 17-alpha-hydroxy-20-oxo-pregnanes from 17(20)-dehydro-23,24-dinorcholan-22-oic acids'
- EXPERIENTIA vol. 18, no. 7 , 15 Juillet 1962 , BASEL CH pages 309 - 310 J. BROWN ET AL 'Steroidal Cyclic Ketals: The Preparation of Steroidal-delta-4, 3-Ethyleneketals'

## Description

La présente invention a pour objet des nouveaux dérivés 17,20-époxydes du pregnane, leur préparation, leur application à la préparation de dérivés cortisoniques et des intermédiaires.

L'invention a ainsi pour objet, les composés de formule (I) : 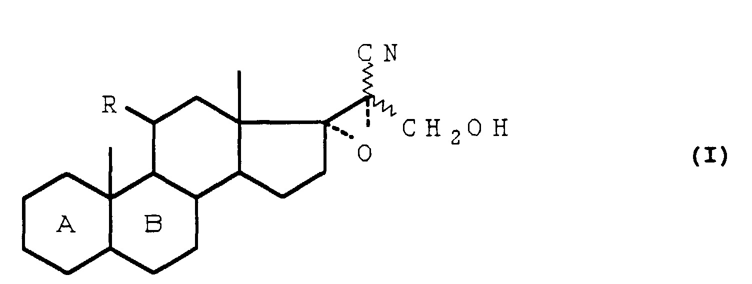 dans laquelle R représente un radical oxo ou β-hydroxy et les cycles A et B représentent un reste : 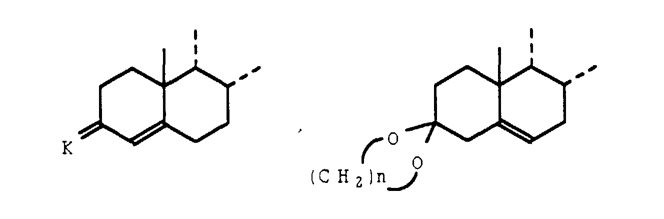 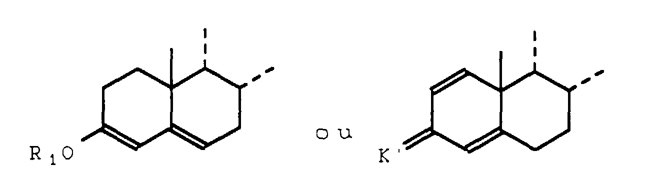 dans lequel K représente un radical oxo ou un groupement protecteur du radical oxo de formule : 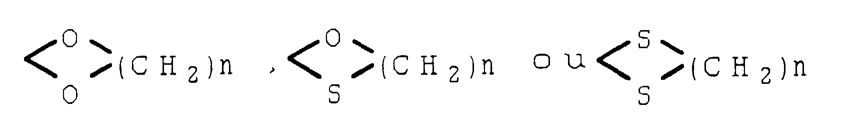 n est égal à 2 ou 3 et R₁ représente un reste éther ou ester, K', représente un radical oxo ou un groupement protecteur de type oxime, hydrazone ou semicarbazone et les traits ondulés symbolisent un mélange d'isomères.

Lorsque R₁ représente un reste éther, il peut s'agir d'un radical alkyle renfermant de 1 à 6 atomes de carbone, d'un radical alkoxyalkoxyalkyle renfermant de 3 à 8 atomes de carbone, d'un radical aryle renfermant de 6 à 10 atomes de carbone ou d'un radical aralkyle renfermant de 7 à 12 atomes de carbone.

Lorsque R₁ représente un radical alkyle, il s'agit par exemple d'un radical méthyle, éthyle, propyle, isopropyle, n-butyle, sec-butyle, tert-butyle, pentyle ou hexyle.

Lorsque R₁ représente un radical alkoxyalkoxyalkyle, il s'agit par exemple d'un radical méthoxyéthoxyméthyle.

Lorsque R₁ représente un radical aralkyle, il s'agit par exemple d'un radical benzyle ou phénéthyle.

Lorsque R₁ représente un radical aryle, il s'agit par exemple d'un radical phényle ou d'un radical phényl substitué, en particulier par un ou plusieurs radicaux alkyles.

Lorsque R₁ représente un reste éther, il peut encore s'agir d'un groupement silylé, par exemple d'un groupement trialkylsilyle tel que triméthylsilyle, tert-butyl, diméthylsilyle ou encore par exemple d'un groupement triarylsilyle tel que triphénylsilyle ou diarylkylsilyle tel que diphényl tert-butylsilyle.

Lorsque R₁ représente un reste ester, il peut s'agir de tout reste connu de l'homme du métier pour bloquer la position 3 sous cette forme et notamment il peut s'agir d'un reste -COR₁, R₁ étant un radical alkyle, aryle ou aralkyle tel que défini ci-dessus.

Lorsque K' représente un groupement protecteur de type oxime ou semicarbazone, il s'agit par exemple d'un reste de formule =N-OX, dans laquelle X représente un atome d'hydrogène, un radical alkyle renfermant de 1 à 6 atomes de carbone, de préférence méthyle, un radical acyle renfermant de 1 à 10 atomes de carbone, de préférence acétyle ou benzoyle ou un radical aryle renfermant de 6 à 12 atomes de carbone, ou d'un reste =N-NH-CO-Y, dans lequel Y représente un radical amino ou un groupement 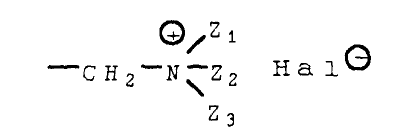 Z₁, Z₂ et Z₃ représentant des radicaux alkyle renfermant de 1 à 6 atomes de carbone ou formant ensemble avec l'atome d'azote un radical pyridyle et Hal représentant un atome d'halogène, de préférence un atome de chlore ou de brome.

L'invention a notamment pour objet les composés tels que définis plus haut, répondant à la formule (I₁) : 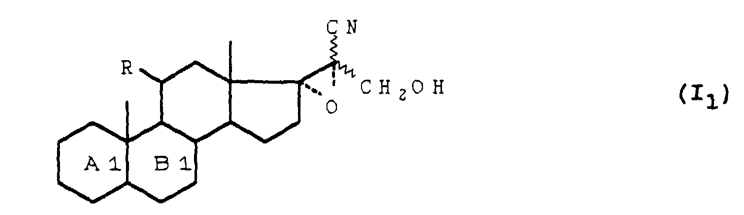 dans laquelle R a la signification déjà indiquée et les cycles A₁ et B₁ représentent un reste : 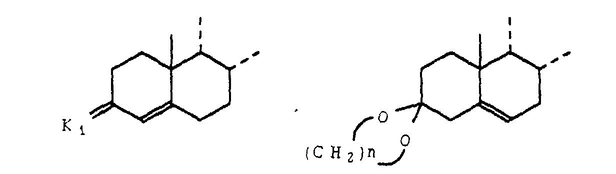 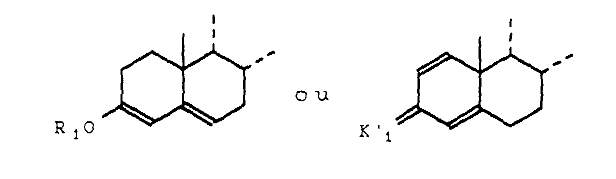 dans lequel n et R₁ ont la signification déjà indiquée et K₁ et K₁' représentent des groupements protecteurs du radical oxo tels que déjà définis et plus particulièrement les composés répondant à la formule (I'₁) : 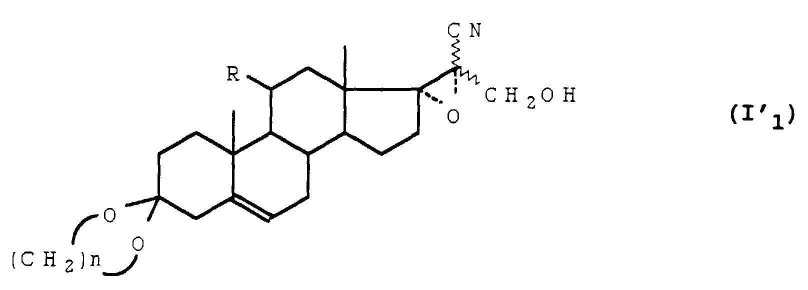 dans laquelle R et n ont les significations déjà indiquées.

L'invention a encore notamment pour objet les composés tels que définis plus haut, répondant à la formule (I₂) : 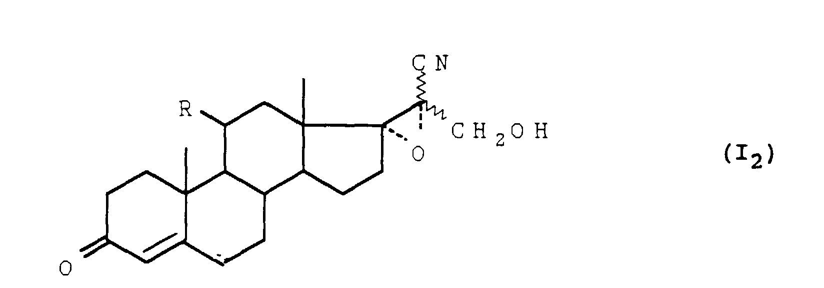 dans laquelle R a la signification déjà indiquée.

L'invention a également pour objet un procédé de préparation des composés de formule (I) telle que défini précédemment, caractérisé en ce que l'on protège la fonction cétone en 3 d'un composé de formule (II) : 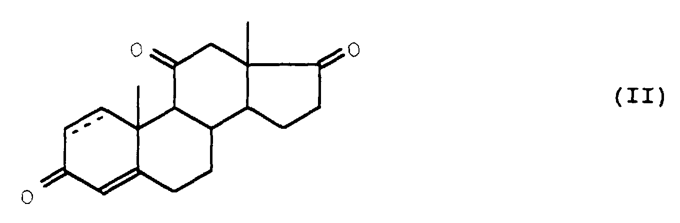 dans laquelle le trait pointillé indique la présence éventuelle d'une seconde liaison pour obtenir un composé de formule (III) : 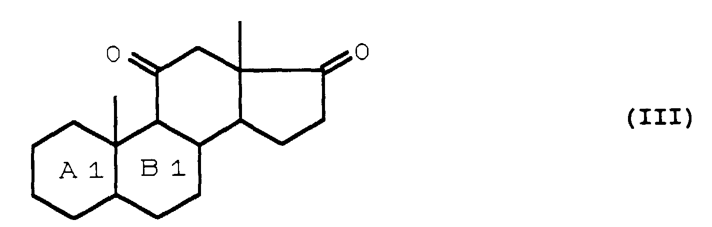 dans laquelle A₁ et B₁ sont définis comme précédemment, que l'on traite par un cyanacétate d'alkyle de formule (IV) :

N≡C-CH₂-CO₂R₂ (IV)

dans laquelle R₂ représente un radical alkyle renfermant de 1 à 6 atomes de carbone, pour obtenir un composé de formule (V) : 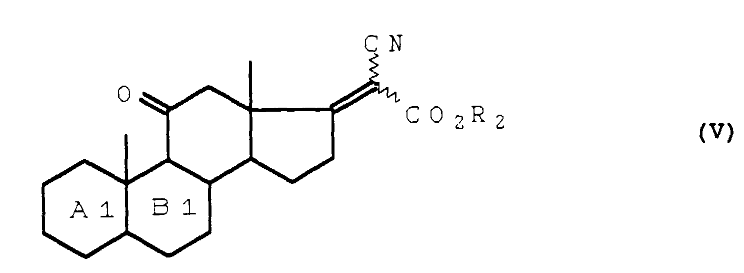 dans laquelle R₂, A₁, B₁ et les traits ondulés sont définis comme précédemment, que l'on traite par un agent d'époxydation, pour obtenir un composé de formule (VI) : 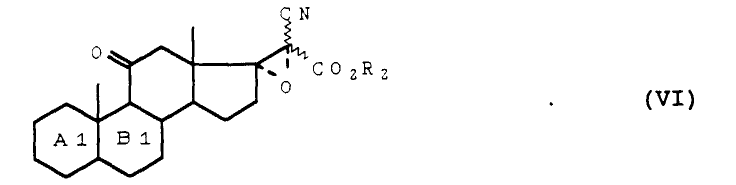 dont on réduit la fonction ester pour obtenir un composé de formule (I₁ₐ) : 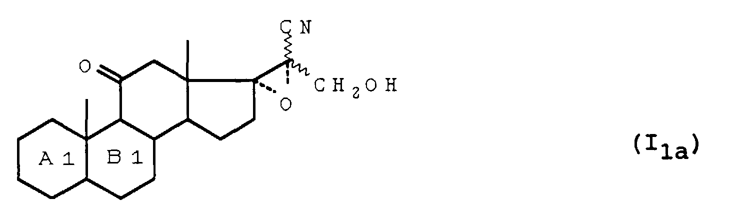 dont on réduit, le cas échéant, la fonction cétone en 11 pour obtenir un composé de formule (I_{1b}) : 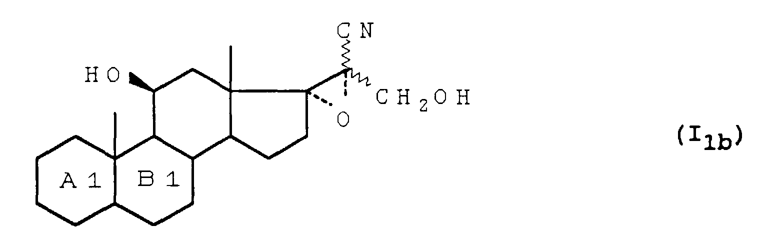 composés de formules (I₁ₐ) et (I_{1b}) dont on libère, le cas échéant, la fonction cétone en 3, pour obtenir un composé 3-céto correspondant.

La protection de la fontion cétone en 3 est effectuée par des méthodes connues de l'homme du métier. On peut ainsi utiliser notamment un diol, dithiol ou un thiol mixte de formule HO-(CH₂)ₙ-OH, HS-(CH₂)ₙ-SH ou HO-(CH₂)ₙ-SH, en milieu acide, par exemple en présence d'acide chlorydrique ou bromhydrique concentré, en quantité catalytique, d'acide P-toluène sulfonique, ou encore en présence d'un acide de Lewis tel que le chlorure de zinc, le tétrachlorure de titane ou le trifluorure de bore de préférence sous forme d'éthérate. On peut encore utiliser le méthyléthyl dioxolane en présence d'un acide, par exemple l'un de ceux nommés ci-dessus.

On peut encore utiliser un halogénure d'alkyle, d'alkoxyalkoxyalkyle, d'aralkyle ou d'aryle, en présence d'une base formant intermédiairement l'énolate, par exemple un hydrure, un alcoolate ou un hydroxyle alcalin.

On peut encore utiliser un halogénure de trialkyl, triaryl ou diarylalkylsilyle en milieu alcalin comme ci-dessus.

On peut encore utiliser un chlorure d'acide approprié, en opérant en présence d'une base, qui peut être une base azotée, par exemple la triéthylamine, la pyridine, la diméthylaminopyridine, ou une base minérale, notamment un hydrure, un alcoolate ou un hydroxyde alcalin.

On peut encore utiliser une hydroxylamine appropriée, éventuellement sous forme de chlorhydrate ou d'un autre sel, ou le semicarbazide ou un dérivé approprié, de même sous forme de chlorhydrate ou d'un autre sel.

L'action du cyanacétate d'alkyle est effectuée de préférence en milieu anhydre en présence d'une amine primaire, par exemple l'hexylamine ou un homologue inférieur et d'un catalyseur acide faible, par exemple un acide de Lewis, une résine acide, l'acide benzoïque ou encore l'acide paratoluène sulfonique.

On opère au sein d'un solvant organique tel qu'un solvant aromatique, par exemple le benzène, le toluène ou le xylène, ou du cyclohexane.

L'agent d'époxydation est un agent qui doit respecter le groupement nitrile. Il est de préférence l'eau oxygénée utilisée seule ou en présence d'un métal de transition tel que le titane, le tungstène ou le molybdène, par exemple sous forme d'un sel hydraté.

On opère de préférence en milieu basique, notamment en présence d'un carbonate, d'un bicarbonate ou d'un hydroxyde alcalin ou alcalino-terreux, au sein d'un solvant qui peut être un alcanol, de préférence en mélange avec un cosolvant, notamment un solvant halogéné.

On peut encore utiliser un hypochlorite alcalin, à pH neutre ou voisin de la neutralité.

Cette réaction d'époxydation d'un nitrile αβ-éthylénique présente un caractère original et inattendu, dans la mesure où le nitrile y est préservé, ce qui a priori, n'est pas évident.

La réduction de la fonction ester est effectuée par action d'un borohydrure alcalin en opérant dans un alcool, au sein d'un solvant organique par exemple le borohydrure de sodium ou de lithium, dans un mélange d'éthanol et de toluène. On peut encore utiliser un hydrure, par exemple l'hydrure double de lithium et d'aluminium, l'hydrure de diéthylsodiumaluminium, l'hydrure de diisobutylaluminium ou encore le dihydrobis(2-méthoxy éthoxy) aluminate de sodium. On opère alors par exemple dans le toluène ou le tétrahydrofuranne.

La réduction de la fonction cétone est effectuée par l'un ou l'autre des réactifs mentionnés ci-dessus, dans les conditions de solvant indiquées.

Il est à noter que les deux réductions s'opèrent dans un ordre inverse à celui que l'on rencontre habituellement, la réduction de l'ester en 21 précédant la réduction de la cétone en 11. Il s'agit là également d'un aspect inattendu du procédé.

Il est évident que l'on peut sans sortir du cadre de l'invention, effectuer les deux réductions en une seule opération, sans isolement de l'intermédiaire 11-oxo. Un tel exemple figure dans la partie expérimentale.

La libération éventuelle de la fonction cétone en 3 est effectuée par des moyens appropriés à la nature du groupement protecteur. On utilise un agent acide en présence d'eau ou d'un mélange eau-alcanol, dans le cas d'un cétal. Il s'agit par exemple d'un acide minéral ou organique tel que l'acide chlorhydrique, bromhydrique, sulfurique, perchlorique, nitrique, paratoluène sulfonique, acétique, formique, oxalique ou d'un mélange d'acides, ou encore d'une résine acide, par exemple une résine sulfonique. Dans le cas d'un thiocétal ou d'un cétal mixte, la déprotection de la fonction 3-oxo est réalisée par action de l'iode en présence d'une base par exemple un bicarbonate alcalin, ou par action de l'iode en quantité catalytique, en présence d'un agent oxydant, notamment l'eau oxygénée, par action de l'iodure de méthyle, de l'acide glyoxylique, ou encore de sels de métaux, tels le mercure ou le cadmium. On peut, en général, opérer dans un solvant tel qu'un alcanol inférieur, par exemple le méthanol ou l'éthanol, en mélange avec un solvant halogéné, par exemple le chlorure de méthylène, en présence d'eau. Dans le cas d'un cétal mixte, la déprotection est encore effectuée par exemple par un sel mercurique tel que le chlorure mercurique en présence d'un tampon acide acétique/acétate de potassium à 100°C environ, par le Nickel de Raney dans les mêmes conditions que ci-dessus ou par le mélange acide chlorhydrique-acide acétique à chaud.

Dans le cas où R₁ représente un reste éther ou ester, on utilise également un traitement acide, notamment dans les conditions décrites ci-dessus pour le cétal. Il en est de même pour la libération de la fonction cétone protégée sous forme d'oxime ou de semicarbazone.

L'invention a également pour objet l'application des composés de formule (I) telle que définie précédemment, à la préparation des composés de formule (A) : 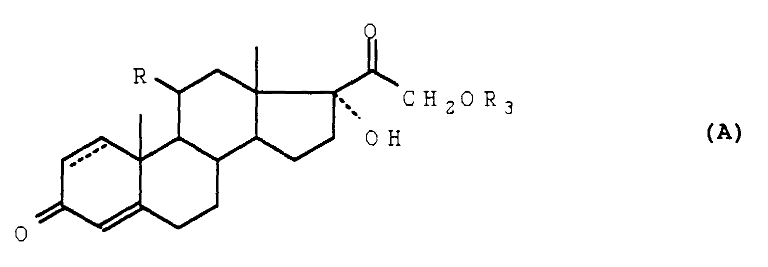 dans laquelle R est défini comme précédemment, R₃ représente un groupement protecteur du radical hydroxy et le trait pointillé représente une éventuelle seconde liaison carbone-carbone, caractérisée en ce que l'on protège la fonction hydroxy en 21 d'un composé de formule (I) , pour obtenir un composé de formule (VII) : 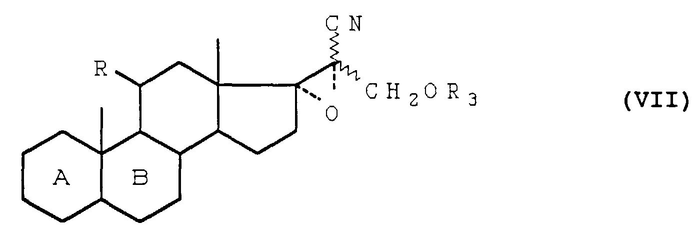 dans laquelle R, R₃, A et B ont la signification déjà indiquée, dont on hydrate la fonction nitrile, pour obtenir un composé de formule (VIII) : 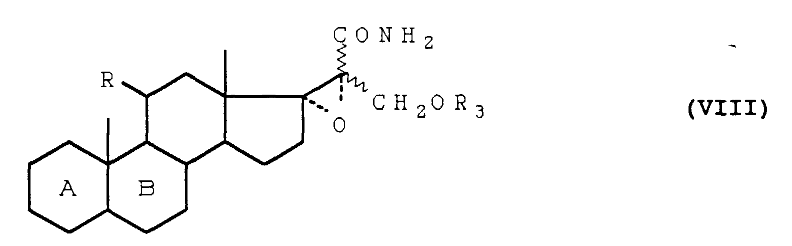 que l'on soumet à une réaction de dégradation d'Hofmann pour obtenir un composé de formule (IX) : 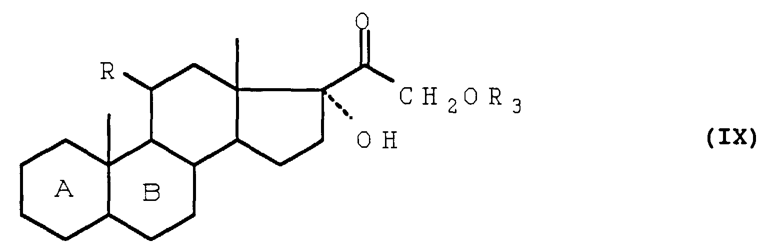 dont on libère, le cas échéant, la fonction cétone en 3, pour obtenir le composé de formule (A) attendu.

Par groupement protecteur du radical hydroxy, on entend un groupement stable dans les conditions du procédé et plus particulièrement de la dégradation d'Hofmann, c'est-à-dire un radical alkyle renfermant de 1 à 6 atomes de carbone, notamment un radical méthyle, éthyle, propyle.

De tels éthers sont préparés notamment par action d'un halogénure correspondant, en présence d'un agent basique, par exemple par action d'un iodure ou d'un bromure approprié, en présence d'une base forte telle qu'un hydrure, un amidure ou un alcoolate alcalin. On opère au sein d'un solvant qui peut notamment être un éther tel que le tétrahydrofuranne ou le dioxanne, le diméthylformamide, le diméthylsulfoxyde.

L'hydratation de la fonction nitrile est effectuée par action d'une base minérale aqueuse et notamment par action d'un hydroxyde alcalin tel que l'hydroxyde de sodium, potassium ou lithium. On opère en présence d'un solvant organique de préférence polaire, tel que le tétrahydrofuranne, le dioxanne, le diméthylformamide, le diméthylsulfoxyde, ou encore l'acétonitrile.

La dégradation de l'amide est effectuée par action d'un hypohalogénite alcalin ou alcalino-terreux, notamment de l'hypochlorite ou de l'hypobromite de sodium, le cas échéant formé in situ. On opère au sein d'un solvant qui est de préférence l'un de ceux mentionnés plus haut, et en présence d'eau.

L'invention s'étend à une application telle que définie plus haut, caractérisée en ce que l'on effectue la dégradation sans isoler intermédiairement l'amide de formule (VIII).

La libération éventuelle de la fonction cétone est effectuée dans les conditions précisées plus haut.

L'invention a enfin pour objet à titre de produits industriels nouveaux :
- les composés de formule : 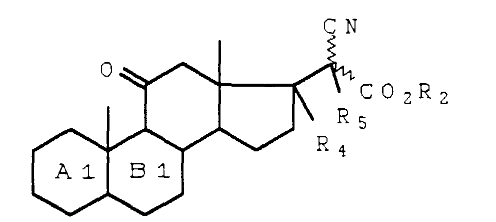 dans laquelle A₁, B₁, R₂ et les traits ondulés sont définis comme précédemment, et R₄ et R₅ forment ensemble une liaison carbone-carbone ou un époxy en position α,
- les composés de formule : 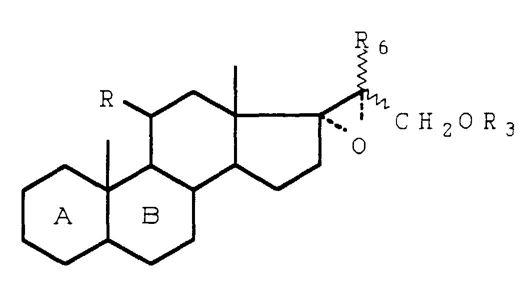 dans laquelle A, B, R, R₃ et les traits ondulés sont définis comme précédemment, et R₆ représente un radical cyano ou un radical carbamoyle.

Le document US-2,813,860 décrit la synthèse des composés actifs du type 17,20- epoxy, 20 cyano 3-2-ol par un procédé différent.

Les composés de formule (A) sont des composés en général connus, par exemple par le brevet US 2 648 688 ou les références J. Org. Chem. 26, 4153-5 (1961) et 26, 5046-52 (1961) soit comme intermédiaires de synthèses, soit comme composés biologiquement actifs.

Le composé de formule (II) est décrit, par exemple, dans le brevet US 2 844 600.

Les exemples suivants illustrent l'invention sans toutefois la limiter.

### EXEMPLE 1 : 3,3-(1,2-éthane diyl) acétal cyclique de 17,20-époxy 20-cyano 21-hydroxy pregn-5-ène 3,11-dione

### STADE A : 3,3-(1,2-éthane diyl) acétal cyclique d'androst 5-ène 3,11,17-trione

On mélange sous gaz inerte 10 g d'adrénostérone et 150 cm³ de méthyléthyldioxolane, porte au reflux pendant 30 mn, ajoute 0,033 g d'acide p-toluène sulfonique et poursuit le reflux pendant 6 h 30 mn. On refroidit vers 50°C, ajoute 0,5 cm³ de triéthylamine et concentre à sec sous pression réduite. On recristallise le produit obtenu dans l'éthanol, on l'essore et on le lave par de l'éthanol froid puis par un mélange éthanol-eau et le sèche. On obtient 6,35 g de produit attendu.
F = 196-198°C
Spectre IR (CHCl₃)
Absorption à 1740 cm⁻¹ (17 C=O) et 1706 cm⁻¹ (11=O).
Spectre RMN (CDCl₃, 250 MHz, ppm)
0,86 : 18-CH₃ ; 3,95 : CH₂ du cétal ; 5,38 : H en 6.

### STADE B : 20-cyano 3,3-(1,2-éthane diyl bis oxy) 11-oxo pregn-5,17(20)-dièn-21-oate d'éthyle

On mélange sous gaz inerte 6,25 g de produit obtenu au stade A, 100 cm³ de xylène, 6,2 cm³ de cyanacétate d'éthyle, 3,1 cm³ d'hexylamine et 0,31 g d'acide benzoïque.

On porte au reflux pendant 24 h en éliminant l'eau formée dans la réaction, puis refroidit vers 15°C et ajoute 5 cm³, d'une solution aqueuse de bicarbonate de sodium à 10 %. On agite pendant 1 h puis décante, lave à l'eau la phase organique et concentre sous pression réduite. On obtient un produit cristallisé que l'on recristallise dans l'acétate d'éthyle. On obtient après lavage des cristaux à l'hexane et séchage 3,42 g de produit attendu. Par chromatographie des liqueurs mères sur silice en éluant au mélange cyclohexane, toluène, acétate d'éthyle (2/2/1), on obtient 2,75 g de produit attendu supplémentaire.
Spectre IR (CHCl₃)
Absorptions à 2226 cm⁻¹ (C≡N), 1725-1707 cm⁻¹ (C=O et ester conjugué), 1611 cm⁻¹ (C=C).
Spectre RMN (CDCl₃, 300 MHz, ppm)
1,01 : CH₃ en 18 ; 1,23 : CH₃ en 19 ; 1,34 (t) : CH₃ de l'ester ; 3,94 : CH₂ de l'acétal, 4,27 (q) : CH₂ de l'ester ; 5,36 : H en 6.

### STADE C : 20-cyano 3,3-(1,2-éthane diyl bis oxy) 11-oxo 17,20-époxy pregn-5-èn-21-oate d'éthyle

On mélange sous gaz inerte 0,255 g de produit obtenu au stade B, 1 cm³ de chlorure de méthylène et 4 cm³ d'éthanol. On ajoute à la solution à 20°C, 0,2 cm³ d'eau oxygénée à 50 %. On ajoute ensuite une solution saturée de bicarbonate de potassium dans de l'éthanol à 50 % jusqu'à pH 9 puis maintient sous agitation à pH constant pendant 3 h 30 mn. On ajoute ensuite 0,5 cm³ d'acide acétique à 10 % puis concentre à sec sous pression réduite à 25°C environ. On reprend le résidu par un mélange d'eau et de chlorure de méthylène, décante et concentre à sec la phase organique. On chromatographie le résidu sur silice en éluant au mélange chlorure de méthylène-acétate d'éthyle (95-5) et obtient 0,236 g de produit attendu.
Spectre IR (CHCl₃)
Absorption à 1764 cm⁻¹ et 1740 cm⁻¹ : carbonyl ; 1707 cm⁻¹ : 11-céto ; 2243-2235 cm⁻¹ : C≡N non conjugué ; 1672 cm⁻¹ : C=C.
Spectre RMN (CDCl₃, 300 MHz, ppm)
1,05 (s) (3H) : 18-CH₃ ; 1,22 (s) (3H) : 19-CH₃ ; 3,95 (m) (4H) : cétal ; 5,30 (m) (1H) : H en 6 ; 1,37 (3H) ; 4,35 (2H) : -CH₂-CH₃-.

### STADE D : 3,3-(1,2-éthane diyl) acétal cyclique de 17,20-époxy 20-cyano 21-hydroxy pregn-5-èn-3,11-dione

On mélange sous gaz inerte 4,36 g de produit obtenu comme décrit au stade C, 44 cm³ de toluène et 44 cm³ d'éthanol. On ajoute à la solution 0,195 g de borohydrure de sodium. On maintient sous agitation pendant 2 h à 20°C puis refroidit à 0°C, ajoute 0,097 g de borohydrure de sodium et poursuit l'agitation pendant 2 h à 0°C. On ajoute ensuite 5 cm³ de chlorure d'ammonium à 20 %, agite pendant 30 mn à 10°C puis concentre à demi volume. On ajoute 50 cm³ d'eau et 50 cm³ d'acétate d'éthyle, agite le mélange puis décante. On sèche la phase organique et la concentre à sec. On chromatographie le résidu sur silice en éluant au mélange chlorure de méthylène-acétate d'éthyle (8-2). On obtient 3,17 g de produit attendu.
Spectre IR (CHCl₃)
Absorptions à 3605 cm⁻¹ (-OH), 2242 cm⁻¹ (-C≡N), 1705 cm⁻¹ (C=O), 1670 cm⁻¹ (C=C).
Spectre RMN (CDCl₃, 200 MHz, ppm)
1,01 : CH₃ en 18 ; 1,22 : CH₃ en 19 ; 3,81 : CH₂ en 21 ; 3,95 : CH₂ de l'acétal ; 5,35 : H en 6.

### EXEMPLE 2 : 3,3-(1,2-éthane diyl) acétal cyclique de 11β,21- dihydroxy 17,20-époxy 20-cyano pregn-5-èn 3-one

On mélange sous gaz inerte 5,9 g de produit obtenu au stade C de l'exemple 1, 120 cm³ d'éthanol et 1,2 g de borohydrure de sodium, puis maintient sous agitation pendant 45 mn à température ambiante. On porte ensuite le mélange à 45°C et on rajoute 0,6 g de borohydrure de sodium, après 1 h 30 mn, puis de nouveau après 3 h. Après 4 h 30 mn, on refroidit à 0-5°C, ajoute lentement 200 cm³ d'une solution saturée de chlorure d'ammonium en maintenant la température réactionnelle inférieure à 15°C. On agite puis introduit un mélange eau-acétate d'éthyle, décante, sèche la phase organique et la concentre à sec. On purifie le produit résiduel par chromatographie sur silice, en éluant au mélange cyclohexane-acétate d'éthyle (1-1) et obtient 3 g de produit attendu.
Spectre IR (CHCl₃)
Absorptions à 3614 cm⁻¹ (OH), 2240 cm⁻¹ (-C≡N), absence de C=O.
Spectre RMN (CDCl₃, 200 MHz, ppm)
1,28 (s) : CH₃ en 18 ; 1,32 (s) : CH₃ en 19 ; 3,76 et 3,87 (d-J = 12) : CH₂ en 21 ; 3,95 : CH₂ du cétal ; 4,5 : H en 11; 5,25 : H en 6.

### EXEMPLE 3 : 17α-hydroxy 21-méthoxy pregn-4-ène 3,11,20-trione

### STADE A : 3,3-(1,2-éthane diyl) acétal cyclique de 17,20-époxy 20-cyano 21-méthoxy pregn-5-ène 3,11-dione

On mélange sous gaz inerte 20 cm³ de tétrahydrofuranne anhydre et 0,668 g d'hydrure de sodium à 50 % dans l'huile. On ajoute en 5 mn sous agitation à 20-22°C, une solution de 4,21 g du produit obtenu à l'exemple 1 dans 30 cm³ de tétrahydrofuranne. On agite pendant 40 mn à 20,22°C, puis ajoute 3 cm³ d'iodure de méthyle et poursuit l'agitation pendant 1 h. On verse la solution ainsi obtenue sur un mélange de 50 cm³ d'une solution aqueuse de chlorure d'ammonium à 20 % et 50 g de glace. On extrait à l'acétate d'éthyle, sèche la phase organique et la concentre à sec. On obtient 4,61 g de produit brut que l'on purifie par empâtage dans un mélange éther isopropylique-acétate d'éthyle. On obtient après séchage 4,03 g de produit attendu. F # 256°C.
Spectre IR (CHCl₃)
Absorptions à 2248 cm⁻¹ (-C≡N), 1706 cm⁻¹ (C=O), 1670-1637 cm⁻¹ (C=C), absence d'OH.
Spectre RMN (CDCl₃, 300 MHz, ppm)
1,00 (s) : CH₃ en 18 ; 1,22 (s) : CH₃ en 19 ; 3,47 (s) : O-CH₃ ; 3,57 : -CH₂ en 21 ; 3,95 : CH₂ du cétal ; 5,35 : H en 6.

### STADE B : 3,3-(1,2-éthane diyl) acétal cyclique de 17,20-époxy 20-carbamoyl 21-méthoxy pregn-5-ène 3,11-dione

On dissout 0,36 g de produit obtenu au stade A et 0,049 g d'hydroxyde de lithium dans 7,5 cm³ de dioxanne et 2,5 cm³ d'eau. On porte au reflux pendant 2 h. On refroidit à température ambiante puis dilue par de l'acide chlorhydrique 0,2 N. On extrait au chlorure de méthylène, sèche la phase organique et concentre à sec. On obtient 0,378 g de produit attendu qui, utilisé tel quel, peut être traité selon la dégradation d'Hofmann comme indiqué dans le stade suivant.
Spectre RMN (CDCl₃, 200 MHz, ppm)
0,88 (s) : CH₃ en 18 ; 1,17 (s) : CH₃ en 19 ; 3,24 (1H,d,J = 11 Hz) et 4,18 (1H,d,J = 11 Hz) : CH₂ en 21 ; 3,39 (3H,s) : O-CH₃ ; 5,37 (1H, dd J = 2,5 Hz et J = 7,5 Hz) : H en 6 ; 5,67 (1H,d, J = 4 Hz) et 6,19 (1H,d, J = 4 Hz) : -NH₂-

### STADE C : 3,3-(1,2-éthane diyl) acétal cyclique de 17α-hydroxy 21-méthoxy pregn-5-ène 3,11,20-trione

On mélange sous gaz inerte 0,36 g de produit obtenu au stade A et 0,049 g d'hydroxyde de lithium. On ajoute 5 cm³ de dioxanne et 1 cm³ d'eau puis porte au reflux pendant 3 h. On refroidit la solution par un bain de glace puis ajoute 0,5 cm³ d'hypochlorite de sodium à 15 % et agite pendant 3 h à 0 +20°C en rajoutant par deux fois 1 cm³ d'hypochlorite de sodium à 15 %. On dilue à l'eau, extrait à l'éther, sèche et concentre à sec la phase organique. On chromatographie le résidu sur silice en éluant au mélange acétate d'éthylecyclohexane (15-85 puis 50-50) et obtient 0,073 g de produit attendu que l'on peut recristalliser dans le mélange chlorure de méthylène-hexane. F = 236°C.
Spectre IR (CHCl₃)
Absorptions à 3610 cm⁻¹ : OH, 1703 cm⁻¹ et 1670 cm⁻¹ : C=O et C=C.
Spectre RMN (CDCl₃, 200 MHz, ppm)
0,63 (s,3H) : CH₃ en 18 ; 1,21 (s, 3H) : CH₃ en 19 ; 3,45 (s, 3H) : O-CH₃ ; 3,95 (4H) : -CH₂ de l'acétal, 4,17 (d, J = 18 Hz, 1H) et 4,36 (d, J = 18 Hz, 1H) : CH₂ en 21, 5,36 (m, 1H): H en 6.

### STADE D : 17α-hydroxy 21-méthoxy pregn-4-ène 3,11,20-trione

On mélange sous gaz inerte 0,036 g de produit obtenu au stade C avec 1 cm³ de méthanol et 0,2 cm³ d'acide chlorhydrique 2N. On maintient sous agitation pendant 4 h, essore le précipité et le sèche. On obtient 0,012 g de produit attendu. Par concentration des liqueurs mères, on obtient à nouveau du produit recherché.
Spectre IR (CHCl₃)
Absorptions à 3610-3480 cm⁻¹ : 17-OH, 1706 cm⁻¹ 11 C=O et 20 C=O ; 1667 cm⁻¹ : 3-C=O conjuguée ; 1617 cm⁻¹ Δ4,5.
Spectre RMN (CDCl₃, 200 MHz, ppm)
0,67 (s) : CH₃ en 18 ; 1,41 (s) : CH₃ en 19 ; 3,44 (s) : O-CH₃ ; 3,47 (s) : OH en 17 ; 4,19 (d) et 4,31 (d) : CH₂ en 21, 5,74 : H en 4.

### EXEMPLE 4 : 17α-hydroxy 21-méthoxy pregn-4-ène 3,11,20-trione

### STADE A : 17,20-époxy 20-cyano 21-hydroxy pregn-4-ène 3,11-dione

On mélange sous gaz inerte 4,1 g de produit obtenu comme décrit à l'exemple 1, 80 cm³ de méthanol et 7 cm³ de chlorure de méthylène. On y ajoute 20 cm³ d'acide chlorhydrique 2N et maintient sous agitation à température ambiante pendant 4 h. On essore les cristaux formés, les lave avec un mélange méthanol-eau (8-2) puis à l'eau. Par concentration des liqueurs mères, on isole du produit supplémentaire. Après séchage, on purifie le produit par chromatographie sur silice en éluant au mélange chloroforme-isopropanol (95-5) et obtient 3,3 g de produit attendu. F = 256°C.
Spectre IR (CHCl₃)
Absorptions à 3310 cm⁻¹ : OH, 1703 et 1654 cm⁻¹ : C=O ; 1612 cm⁻¹ : C=C.
Spectre RMN (CDCl₃, 300 MHz, ppm)
1,04 (s) : CH₃ en 18 ; 1,42 (s) : CH₃ en 19 ; 3,84 (syst AB): -CH₂OH ; 5,74 (s) : H en 4.

| Analyse : C₂₂H₂₇O₄N (369,52) | | | |
|---|---|---|---|
| | C % | H % | N % |
| Calculé | 71,4 | 7,3 | 3,8 |
| Trouvé | 71,3 | 7,3 | 3,5 |

### STADE B : 17,20-époxy 20-cyano 21-méthoxy pregn-4-ène 3,11-dione

On prépare l'éther en 21 selon le procédé décrit au stade A de l'exemple 3, au départ du produit obtenu au stade A ci-dessus.

### STADE C : 17α-hydroxy 21-méthoxy pregn-4-ène 3,11,20-trione

On opère au départ du composé obtenu au stade B ci-dessus, soit comme indiqué au stade B de l'exemple 3 en isolant intermédiairement la 17,20-époxy 20-carbamoyl 21-méthoxy pregn-4-ène 3,11-dione, puis en traitant ce composé par l'eau de javel selon le principe de la dégradation d'Hofmann, soit comme indiqué au stade C de l'exemple 3, c'est-à-dire en opérant cette dégradation sans isoler intermédiairement le composé 20-carbamoyl. On obtient le produit attendu, identique à celui obtenu au stade D de l'exemple 3.

### EXEMPLE 5 : 11β,17α-dihydroxy 21-méthoxy pregn-4-ène 3,20-dione

### STADE A : 3,3-(1,2-éthane diyl) acétal cyclique de 11β-hydroxy 17,20-époxy 20-cyano 21-méthoxy pregn-5-èn 3-one

On opère comme indiqué au stade A de l'exemple 3, au départ du produit obtenu à l'exemple 2 et obtient le produit recherché.

### STADE B : 3,3-(1,2-éthane diyl) acétal cyclique de 11β,17α-dihydroxy 21-méthoxy pregn-5-ène 3,20-dione

On opère au départ du produit obtenu au stade A ci-dessus, soit comme indiqué au stade B de l'exemple 3 en isolant intermédiairement la 3,3-(1,2-éthane diyl) acétal cyclique de 11β-hydroxy 17,20-époxy 20-carbamoyl 21-méthoxy preg-5-èn-3-one, puis en traitant ce composé par l'eau de javel selon le principe de la dégradation d'Hofmann, soit comme indiqué au stade C de l'exemple 3, c'est-à-dire en opérant cette dégradation sans isoler intermédiairement le composé 20-carbamoyle.

### STADE C : 11β,17α-dihydroxy 21-méthoxy pregn-4-ène 3,20-dione

On opère comme indiqué au stade D de l'exemple 3, au départ du composé obtenu au stade B ci-dessus. On obtient le produit attendu.

### EXEMPLE 6: 11β,17α-dihydroxy 21-méthoxy pregn-4-ène 3,20-dione

### STADE A : 11β,21-dihydroxy 17,20-époxy 20-cyano pregn-4-èn 3-one

On mélange sous gaz inerte 3 g de produit obtenu à l'exemple 2, 66 cm³ de méthanol et 10 cm³ de chlorure de méthylène. On ajoute 15 cm³ d'acide chlorhydrique 2N et maintient sous agitation à température ambiante pendant 2 h. On essore les cristaux, on les lave au mélange méthanol-eau (75-25) puis à l'eau, essore les cristaux qui ont précipité, les lave à l'eau puis enfin concentre les liqueurs mères, essore de nouveau les cristaux formés et les lave à l'eau. On sèche l'ensemble des cristaux et les recristallise dans le méthanol. On obtient 1 g de produit attendu.
Spectre IR (CHCl₃)
Absorptions à 3485 cm⁻¹ : région OH/NH, 2240 cm⁻¹ : C≡N, 1628 et 1610 cm⁻¹ : C=O conjuguée.
Spectre RMN (CDCl₃, 300 MHz, ppm)
1,34 (s) : CH₃ en 18 ; 1,50 (s) : CH₃ en 19 ; 3,81 (d) : OH en 11 ; 3,76 et 3,88 (syst AB) : CH₂ en 21 ; 4,45 : H en 11 ; 5,69 (s) : H en 4 ; 7,18 : H mobile.

| Analyse : C₂₂H₂₉O₄N (371,52) | | | |
|---|---|---|---|
| | C % | H % | N % |
| Calculé | 71,12 | 7,86 | 3,77 |
| Trouvé | 71,00 | 7,90 | 3,70 |

### STADE B : 11β-hydroxy 17,20-époxy 20-cyano 21-méthoxy pregn-4-èn 3-one

On prépare l'éther en 21 selon le procédé décrit au stade A de l'exemple 3, au départ du produit obtenu au stade A ci-dessus.

### STADE C : 11β,17α-dihydroxy 21-méthoxy pregn-4-èn 3,20-dione

On opère au départ du produit obtenu au stade B ci-dessus, soit comme indiqué au stade B de l'exemple 3 en isolant intermédiairement la 11β-hydroxy 17,20-époxy 20-carbamoyl 21-méthoxy pregn-4-èn 3-one puis en traitant ce composé par l'eau de javel selon le principe de la dégradation d'Hofmann, soit comme indiqué au stade C de l'exemple 3, c'est-à-dire en opérant cette dégradation sans isoler intermédiairement le produit 20-carbamoyl. On obtient le produit attendu, identique à celui obtenu au stade C de l'exemple 5.

## Revendications

1. Les composés de formule (I) : 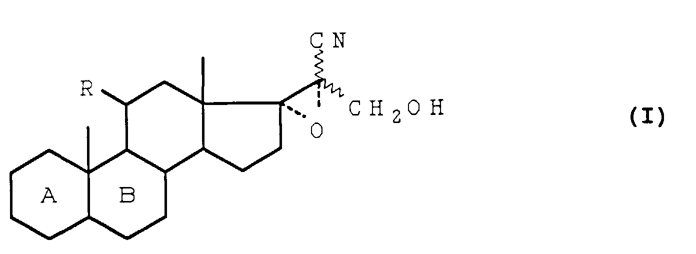 dans laquelle R représente un radical oxo ou β-hydroxy et les cycles A et B représentent un reste : 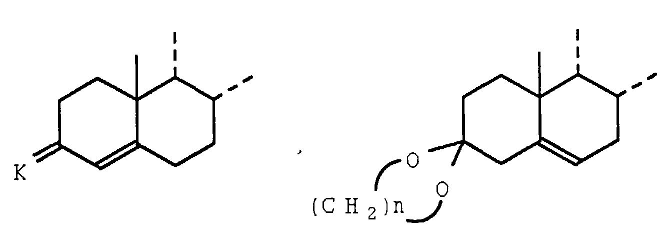 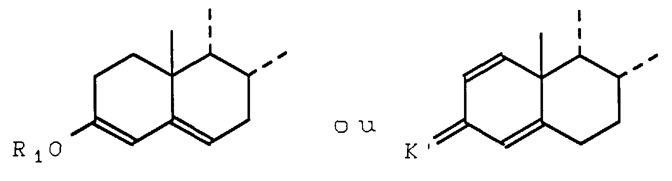 dans lequel K représente un radical oxo ou un groupement protecteur du radical oxo de formule : 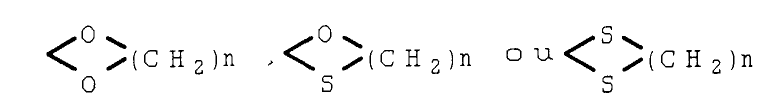 n est égal à 2 ou 3 et R₁ représente un reste éther ou ester choisi dans le groupe constitué par les radicaux alkyles renfermant de 1 à 6 atomes de carbone, alkoxy alkoxy alkyle renfermant de 3 à 8 atomes de carbone, aryle renfermant de 6 à 10 atomes de carbone, aralkyle renfermant de 7 à 12 atomes de carbone, trialkylsilyle, triarylsilyle ou diarylalkyl silyle et COR₁, R₁ ayant les valeurs alkyle, aryle ou aralkyle ci-dessus, K' représente un radical oxo ou un groupement protecteur de type oxime, hydrazone ou semicarbazone choisi dans le groupe constitué par les radicaux =N-OX dans lesquels X représente un hydrogène, un alkyle renfermant de 1 à 6 atomes de carbone, acyle renfermant de 1 à 10 atomes de carbone ou aryle renfermant de 6 à 12 atomes de carbone, et =N-NH-CO-Y dans lesquels Y représente un radical amino ou un groupement 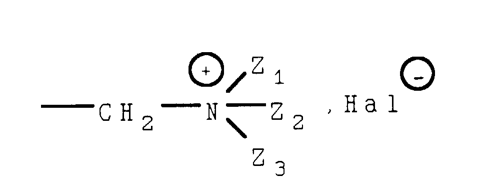 dans lequel Z₁, Z₂ et Z₃ représentent des radicaux alkyles renfermant de 1 à 6 atomes de carbone ou forment ensemble avec l'atome d'azote un radical piridyle et Hal représente un atome d'halogène et les traits ondulés symbolisent un mélange d'isomères.

2. Les composés selon la revendication 1, répondant à la formule (I₁) : 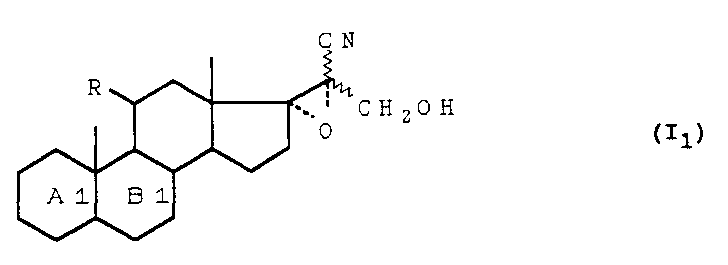 dans laquelle R a la signification déjà indiquée et les cycles A₁ et B₁ représentent un reste : 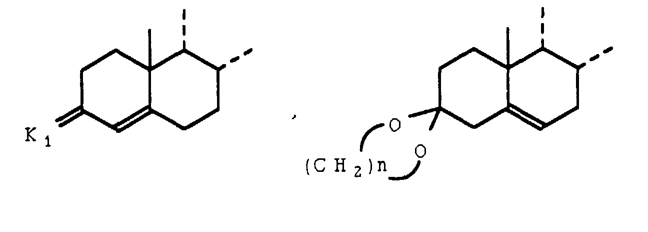 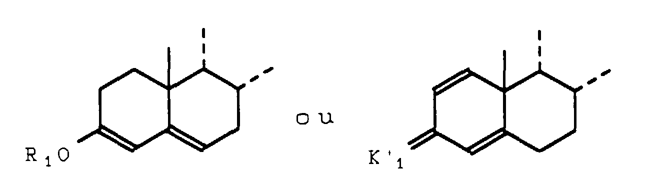 dans lequel n et R₁ ont la signification déjà indiquée et K₁ et K'₁ représentent des groupements protecteurs du radical oxo tels que déjà définis.

3. Les composés selon la revendication 1 ou 2, répondant à la formule (I'₁) : 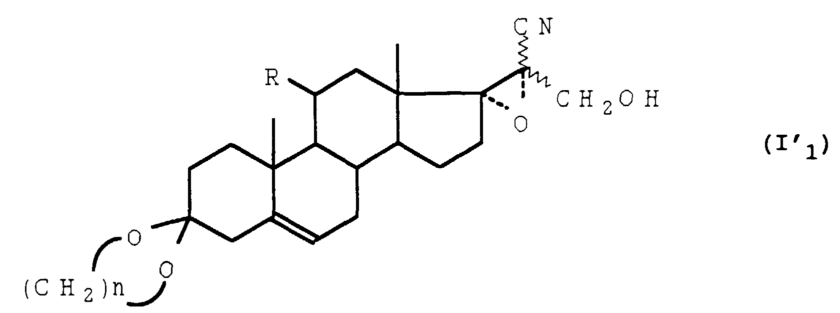 dans laquelle R et n ont les significations déjà indiquées.

4. Les composés selon la revendication 1, répondant à la formule (I₂) : 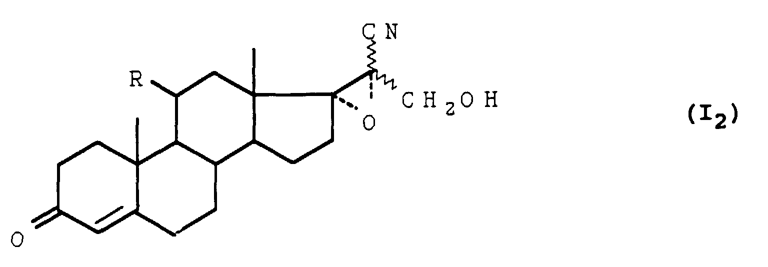 dans laquelle R a la signification déjà indiquée.

5. Procédé de préparation des composés de formule (I) telle que défini à la revendication 1, caractérisé en ce que l'on protège la fonction cétone en 3 d'un composé de formule (II): 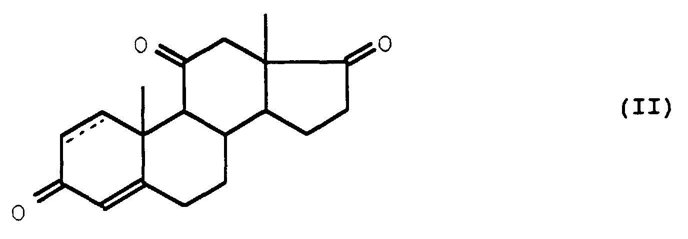 dans laquelle le trait pointillé indique la présence éventuelle d'une seconde liaison pour obtenir un composé de formule (III) : 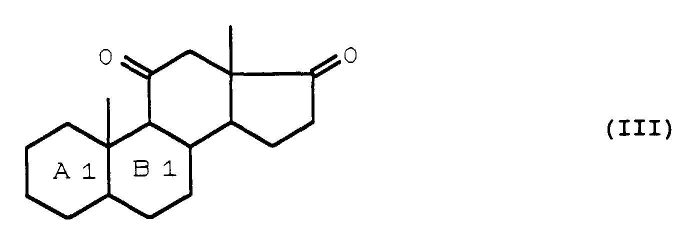 dans laquelle A₁ et B₁ sont définis comme précédemment, que l'on traite par un cyanacétate d'alkyle de formule (IV) :
N≡C-CH₂-CO₂R₂ (IV)
dans laquelle R₂ représente un radical alkyle renfermant de 1 à 6 atomes de carbone, pour obtenir un composé de formule (V) : 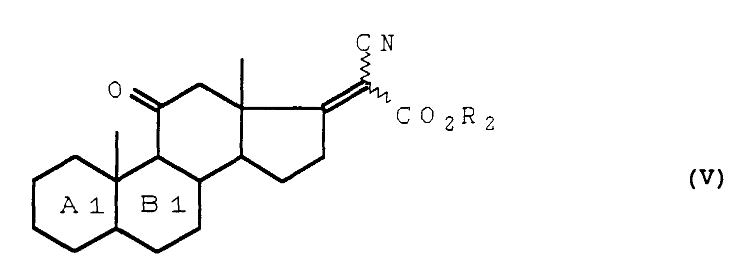 dans laquelle R₂, A₁, B₁ et les traits ondulés sont définis comme précédemment, que l'on traite par un agent d'époxydation, pour obtenir un composé de formule (VI) : 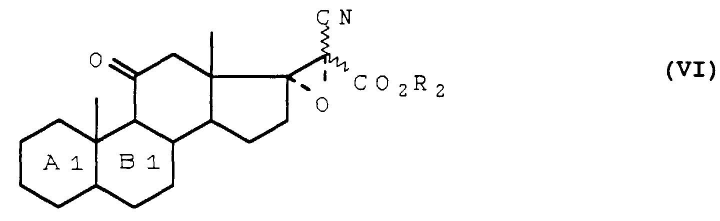 dont on réduit la fonction ester pour obtenir un composé de formule (I₁ₐ) : 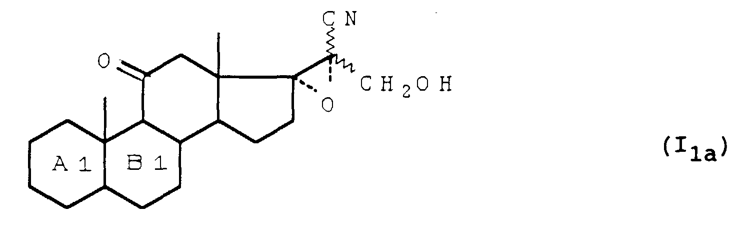 dont on réduit, le cas échéant, la fonction cétone en 11 pour obtenir un composé de formule (I_{1b}) : 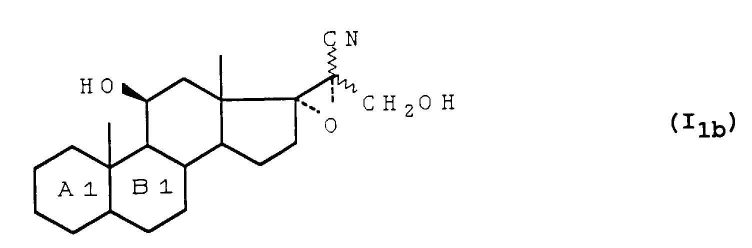 composés de formules (I₁ₐ) et (I_{1b}) dont on libère, le cas échéant, la fonction cétone en 3, pour obtenir un composé 3-céto correspondant.

6. Procédé selon la revendication 5, caractérisé en ce que :
- l'on protège la fonction cétone en 3 sous forme d'alkylène cétal ;
- l'agent d'époxydation est l'eau oxygénée et l'on opère en milieu basique ;
- l'agent de réduction de la fonction ester et de la fonction cétone est un borohydrure alcalin.

7. Application des composés de formule (I), telle que définie à la revendication 1, à la préparation des composés de formule (A) : 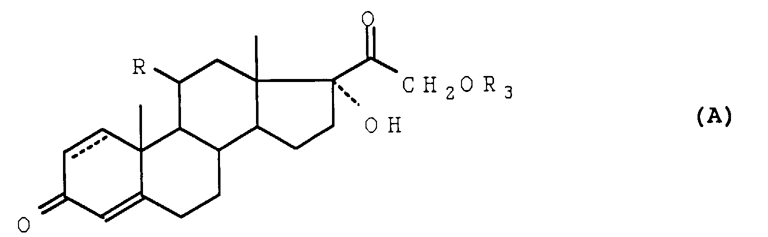 dans laquelle R est défini comme précédemment, R₃ représente un radical alkyle renfermant de 1 à 6 atomes de carbone et le trait pointillé représente une éventuelle seconde liaison carbone-carbone, caractérisée en ce que l'on protège la fonction hydroxy en 21 d'un composé de formule (I), pour obtenir un composé de formule (VII) : 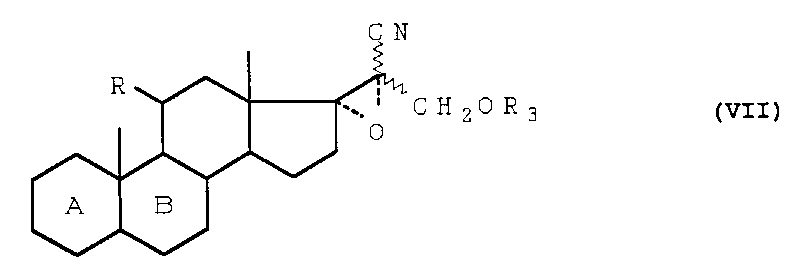 dans laquelle R, R₃, A et B ont la signification déjà indiquée, dont on hydrate la fonction nitrile, pour obtenir un composé de formule (VIII) : 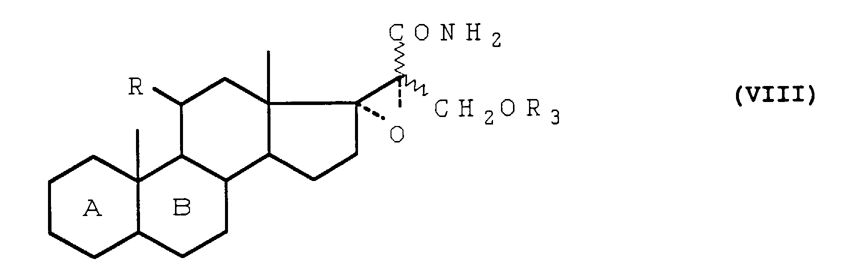 que l'on soumet à l'action d'un hypohalogénite alcalin ou alcalino-terreux au sein d'un solvant organique polaire aqueux pour obtenir un composé de formule (IX) : 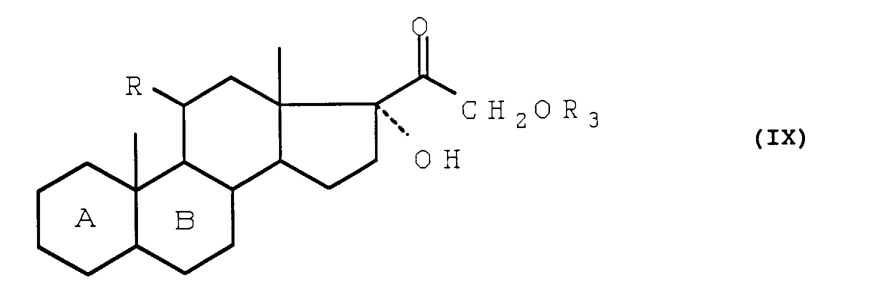 dont on libère, le cas échéant, la fonction cétone en 3, pour obtenir le composé de formule (A) attendu.

8. Application selon la revendication 7, caractérisée en ce que l'on effectue la dégradation sans isoler intermédiairement l'amide de formule (VIII).

9. Application selon l'une quelconque des revendications 7 et 8, caractérisée en ce que l'hydratation de la fonction nitrile est effectuée par action d'une base minérale aqueuse, au sein d'un solvant polaire.

10. Les composés de formule : 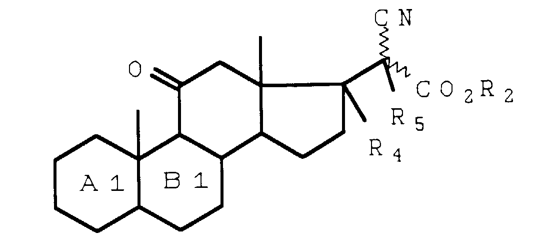 dans laquelle A₁, B₁, R₂ et les traits ondulés sont définis comme à la revendication 5, et R₄ et R₅ forment ensemble une liaison carbone-carbone ou un époxy en position α.

11. Les composés de formule : 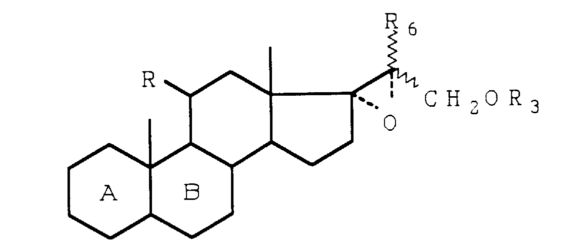 dans laquelle A, B, R et les traits ondulés sont définis comme à la revendication 1, R₃ est défini comme à la revendication 7 et R₆ représente un radical cyano ou un radical carbamoyle.

## Patentansprüche

1. Verbindungen der Formel (I): 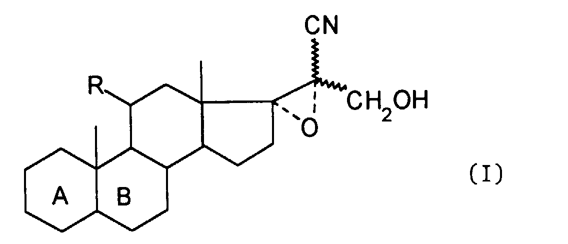 worin R eine Oxo- oder β-Hydroxygruppe bedeutet und die Ringe A und B einen Rest: 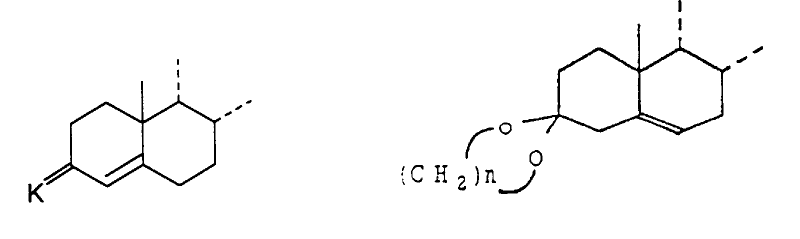 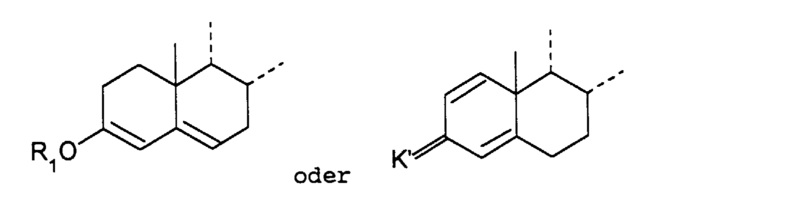 bedeuten, worin K eine Oxogruppe oder eine Schutzgruppe der Oxogruppe der Formel 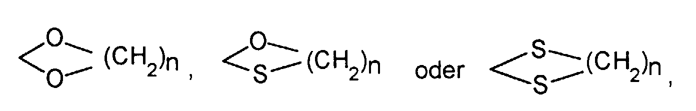 bedeutet, worin n 2 oder 3 bedeutet und R₁ einen Ether- oder Esterrest, ausgewählt aus der Gruppe, bestehend aus Alkylresten mit 1 bis 6 Kohlenstoffatomen, Alkoxyalkoxyalkylresten mit 3 bis 8 Kohlenstoffatomen, Arylresten mit 6 bis 10 Kohlenstoffatomen, Aralkylresten mit 7 bis 12 Kohlenstoffatomen, Trialkylsilyl-, Triarylsilyl- oder Diarylalkylsilylresten und COR_{1,} bedeutet, worin R₁ die vorstehenden Alkyl-, Aryl- oder Aralkylreste bedeutet, K' eine Oxogruppe oder eine Schutzgruppe vom Oxim-, Hydrazon- oder Semicarbazontyp, ausgewählt aus der Gruppe, bestehend aus den Resten =N-OX, worin X ein Wasserstoffatom, einen Alkylrest mit 1 bis 6 Kohlenstoffatomen, einen Acylrest mit 1 bis 10 Kohlenstoffatomen oder einen Arylrest mit 6 bis 12 Kohlenstoffatomen bedeutet, und =N-NH-CO-Y, worin Y eine Aminogruppe oder eine Gruppierung 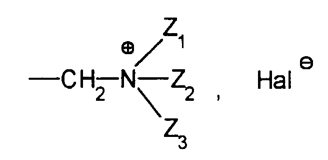 bedeutet, worin Z₁, Z₂ und Z₃ Alkylreste mit 1 bis 6 Kohlenstoffatomen bedeuten oder zusammen mit dem Stickstoffatom einen Piridylrest bilden und Hal ein Halogenatom bedeutet, bedeutet, und die Wellenlinien ein Isomerengemisch bedeuten.

2. Verbindungen nach Anspruch 1 der Formel (I₁): 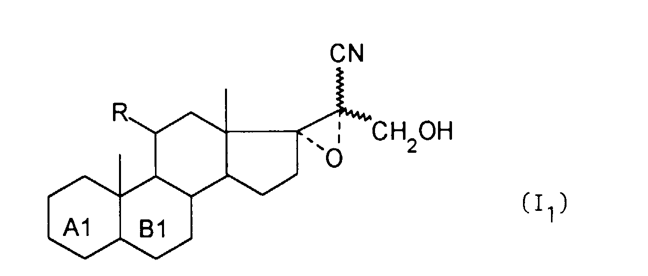 worin R wie vorstehend definiert ist und die Ringe A₁ und B₁ einen Rest 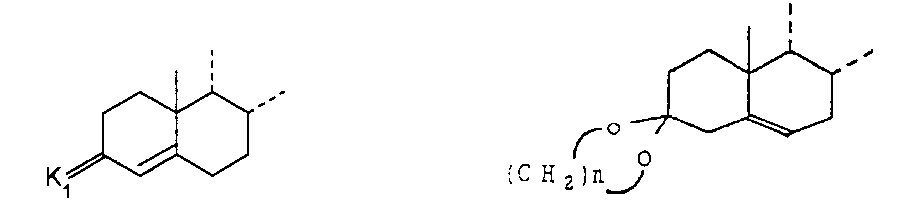 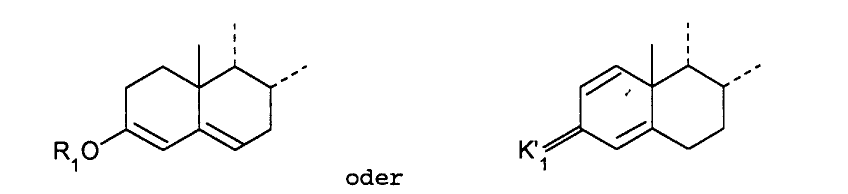 bedeuten, worin n und R₁ wie vorstehend definiert sind und K₁ und K'₁ Schutzgruppen des Oxorestes wie vorstehend definiert, bedeuten.

3. Verbindungen nach Anspruch 1 oder 2 der Formel (I'₁) 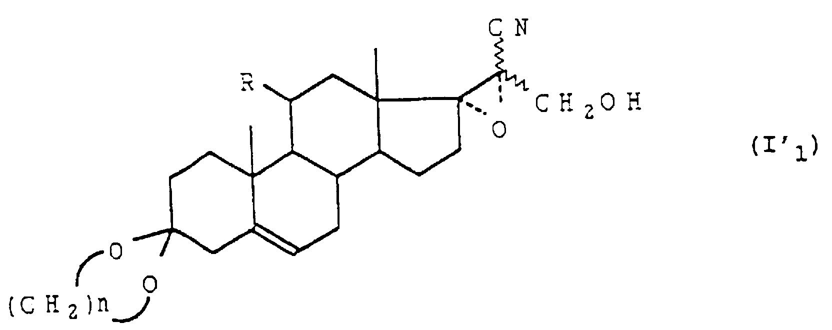 worin R und n wie vorstehend definiert sind.

4. Verbindungen nach Anspruch 1 der Formel (I₂) 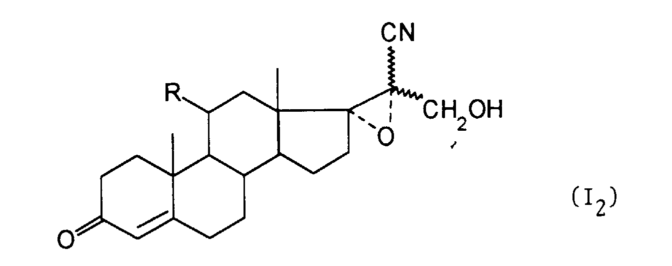 worin R wie vorstehend definiert ist.

5. Verfahren zur Herstellung der Verbindungen der Formel (I), wie in Anspruch 1 definiert, dadurch **gekennzeichnet**, daß man die Ketonfunktion in 3 -Stellung eine Verbindung der Formel (II): 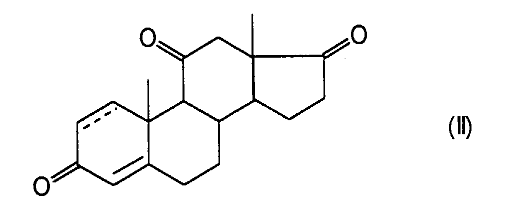 worin die punktierte Linie die fakultative Anwesenheit einer zweiten Bindung bedeutet, schützt, um eine Verbindung der Formel (III) zu erhalten: 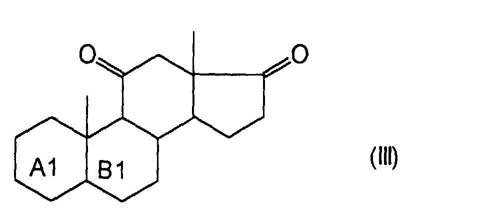 worin A₁ und B₁ wie vorstehend definiert sind, die man mit einem Alkylcyanacetat der Formel (IV):
N ≡ C - CH₂ - CO₂R₂ (IV)
worin R₂ einen Alkylrest mit 1 bis 6 Kohlenstoffatomen bedeutet, behandelt, um eine Verbindung der Formel (V): 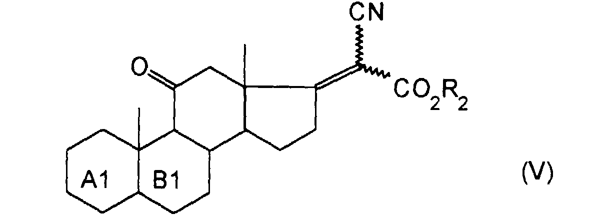 zu erhalten, worin R₂, A₁, B₁ und die Wellenlinien wie vorstehend definiert sind, man die Verbindung mit einem Epoxidierungsmittel behandelt, um eine Verbindung der Formel (VI) 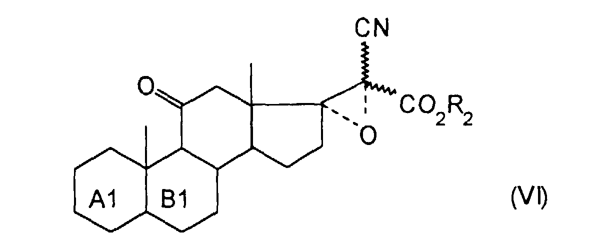 zu erhalten, deren Esterfunktion man reduziert, um eine Verbindung der Formel (I₁ₐ) 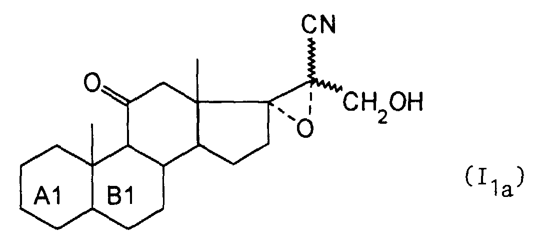 zu erhalten, deren Ketonfunktion in Position 11 man ggf. reduziert, um eine Verbindung der Formel (I_{1b}) 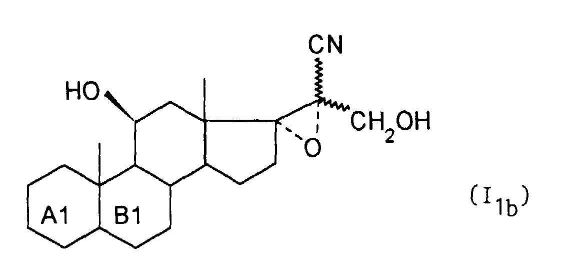 zu erhalten, wobei man in den Verbindungen der Formeln (I₁ₐ) und (I_{1b}) ggf. die Ketonfunktion in Position 3 freisetzt, um eine entsprechende 3-Ketoverbindung zu erhalten.

6. Verfahren nach Anspruch 5, dadurch **gekennzeichnet**, daß man
- die Ketonfunktion in Position 3 in Form eines Alkylenketals schützt;
- das Epoxidierungsmittel oxigeniertes Wasser ist und man in einem basischen Medium arbeitet;
- das Reduktionsmittel der Esterfunktion und der Ketonfunktion ein Alkaliborhydrid ist.

7. Verwendung der Verbindungen der Formel (I), wie in Anspruch 1 definiert, zur Herstellung von Verbindungen der Formel (A) 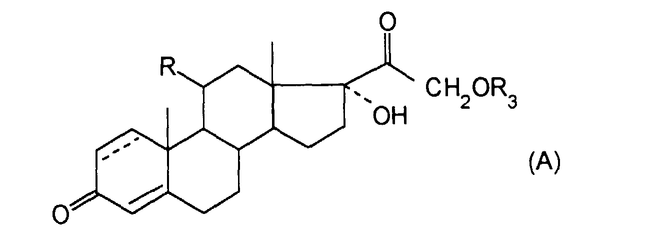 worin R wie vorstehend definiert ist, R₃ einen Alkylrest mit 1 bis 6 Kohlenstoffatomen bedeutet und die punktierte Linie eine fakultative zweite Kohlenstoff-Kohlenstoff-Bindung bezeichnet, dadurch **gekennzeichnet**, daß man die Hydroxyfunktion in Position 21 einer Verbindung der Formel (I) schützt, um eine Verbindung der Formel (VII) 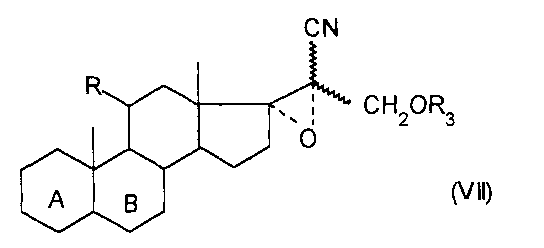 zu erhalten, worin R, R₃, A und B wie vorstehend definiert sind, wobei man die Nitrilfunktion hydratisiert, um eine Verbindung der Formel (VIII) 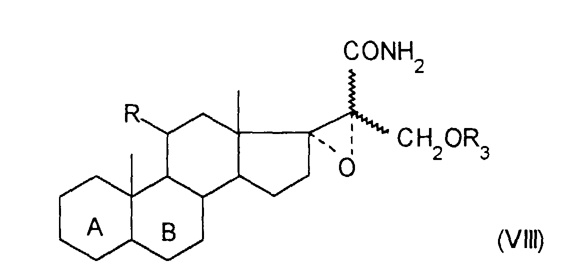 zu erhalten, die man mit einem Alkali- oder Erdalkalihypohalogenit in einem wäßrigen polaren organischen Lösungsmittel umsetzt, um eine Verbindung der Formel (IX) 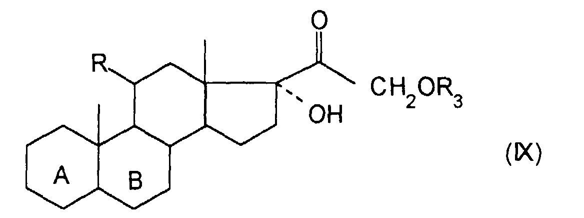 zu erhalten, deren Ketonfunktion in Position 3 man ggf. freisetzt, um die erwartete Verbindung der Formel (A) zu erhalten.

8. Verwendung nach Anspruch 7, dadurch **gekennzeichnet**, daß man den Abbau durchführt, ohne das Amid der Formel (VIII) als Zwischenprodukt zu isolieren.

9. Verwendung nach einem der Ansprüche 7 und 8, dadurch **gekennzeichnet**, daß man die Hydratisierung der Nitrilfunktion mit einer wäßrigen mineralischen Base in einem polaren Lösungsmittel durchführt.

10. Verbindungen der Formel 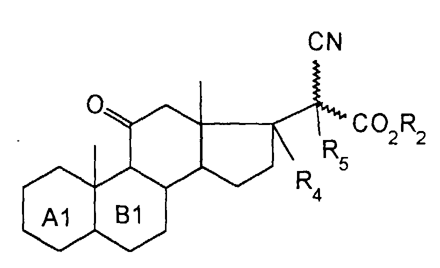 worin A₁, B₁, R₂ und die Wellenlinien wie in Anspruch 5 definiert sind und R₄ und R₅ zusammen eine Kohlenstoff-Kohlenstoff-Bindung oder eine Epoxygruppe in α-Position bilden.

11. Verbindungen der Formel 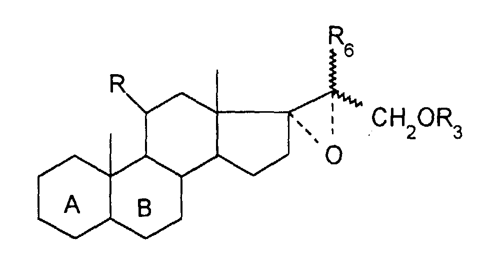 worin A, B, R und die Wellenlinien wie in Anspruch 1 definiert sind, R₃ wie in Anspruch 7 definiert ist und R₆ einen Cyano- oder Carbamoylrest bedeutet.

## Claims

1. The compounds of formula (I): 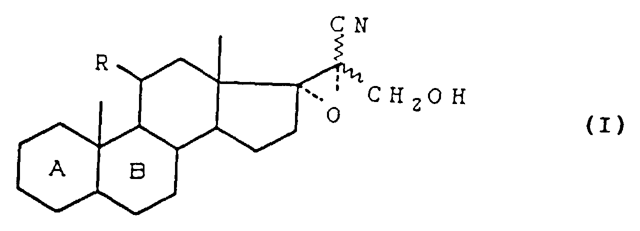 in which R represents an oxo or beta-hydroxy radical and rings A and B represent a remainder: 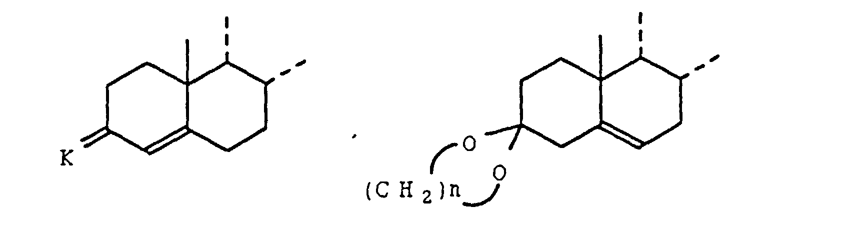 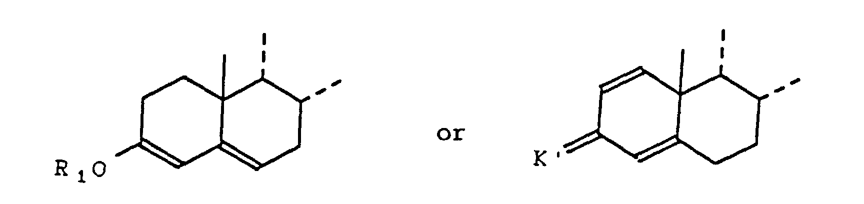 in which K represents an oxo radical or a protector group of the oxo radical of formula: 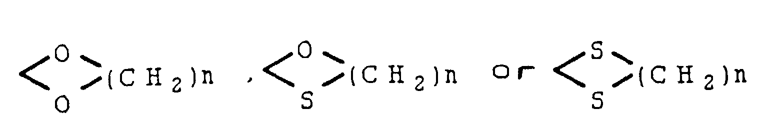 n is equal to 2 or 3 and R₁ represents an ether or ester remainder chosen from the group constituted by the following radicals: alkyl containing 1 to 6 carbon atoms, alkoxy alkoxy alkyl containing 3 to 8 carbon atoms, aryl containing 6 to 10 carbon atoms, aralkyl containing 7 to 12 carbon atoms, trialkylsilyl, triarylsilyl or diarylalkylsilyl and COR₁, R₁ having the above alkyl, aryl or aralkyl values, K' represents an oxo radical or a protector group of oxime, hydrazone or semicarbazone type chosen from the group constituted by the =N-OX radicals in which X represents a hydrogen, an alkyl radical containing 1 to 6 carbon atoms, an acyl radical containing 1 to 10 carbon atoms or an aryl radical containing 6 to 12 carbon atoms, and =N-NH-CO-Y in which Y represents an amino radical or a group 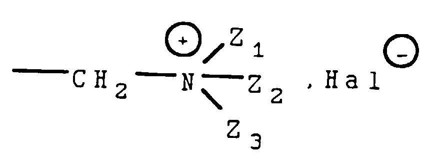 in which Z₁, Z₂ and Z₃ represent alkyl radicals containing 1 to 6 carbon atoms or form together with the nitrogen atom a pyridyl radical and Hal represents a halogen atom and the wavy lines symbolize an isomer mixture.

2. The compounds according to claim 1, corresponding to formula (I₁): 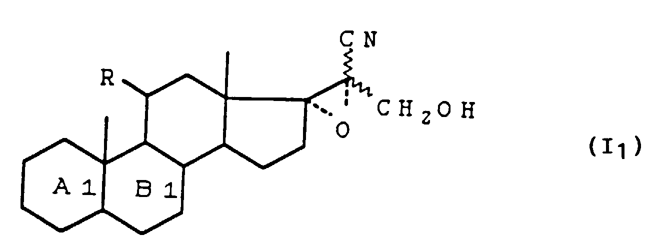 in which R has the meaning already indicated and rings A₁ and B₁ represent a remainder: 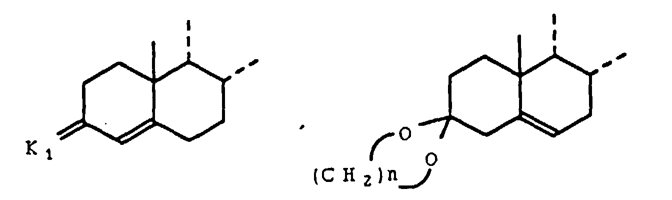 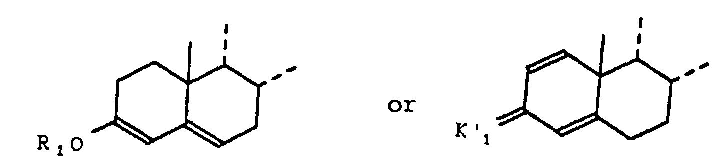 in which n and R₁ have the meaning already indicated and K₁ and K'₁ represent protector groups of the oxo radical as already defined.

3. The compounds according to claim 1 or 2, corresponding to formula (I'₁): 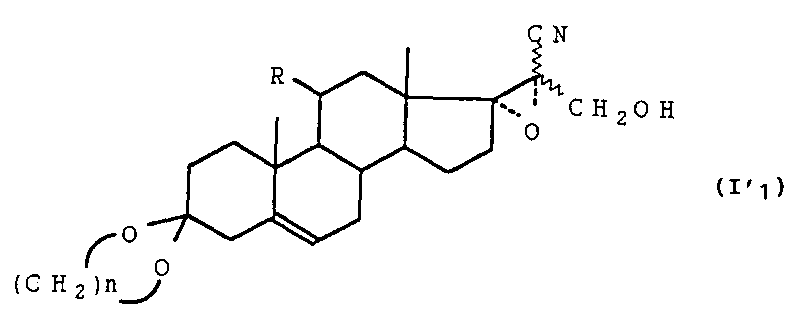 in which R and n have the meanings already indicated.

4. The compounds according to claim 1, corresponding to formula (I₂): 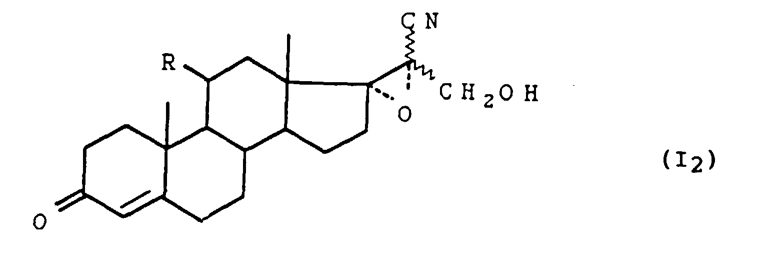 in which R has the meaning already indicated.

5. Preparation process for the compounds of formula (I) as defined in claim 1, characterized in that the ketone function in position 3 of a compound of formula (II): 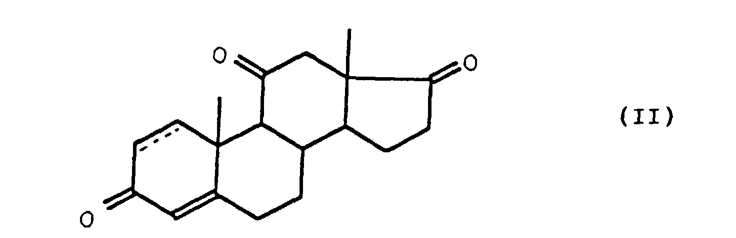 in which the dotted line indicates the optional presence of a second bond, is protected, in order to obtain a compound of formula (III): 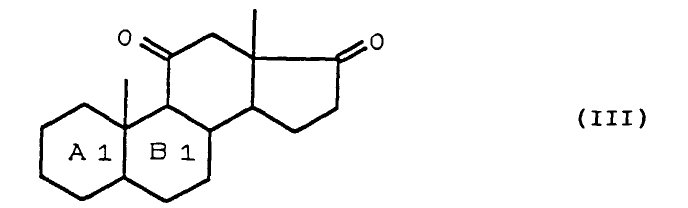 in which A₁ and B₁ are defined as previously, which is treated with an alkyl cyanoacetate of formula (IV):
N≡C-CH₂-CO₂R₂ (IV)
in which R₂ represents an alkyl radical containing 1 to 6 carbon atoms, in order to obtain a compound of formula (V): 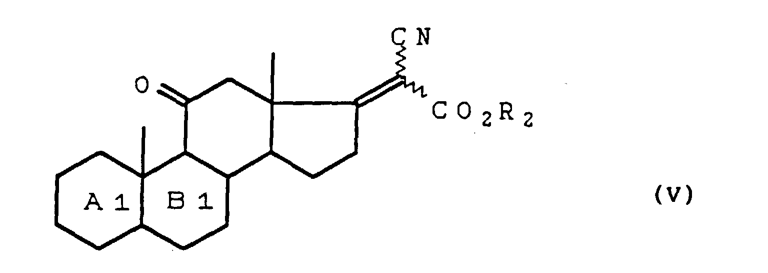 in which R₂, A₁, B₁ and the wavy lines are defined as previously, which is treated with an epoxidation agent, in order to obtain a compound of formula (VI): 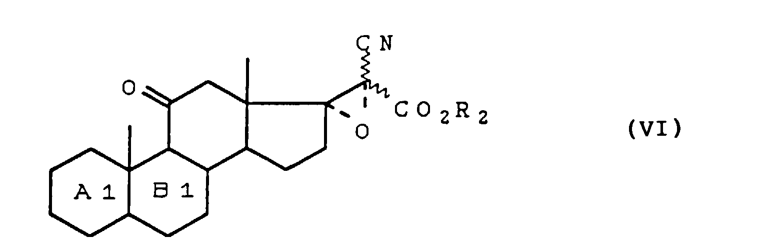 the ester function of which is reduced in order to obtain a compound of formula (I₁ₐ): 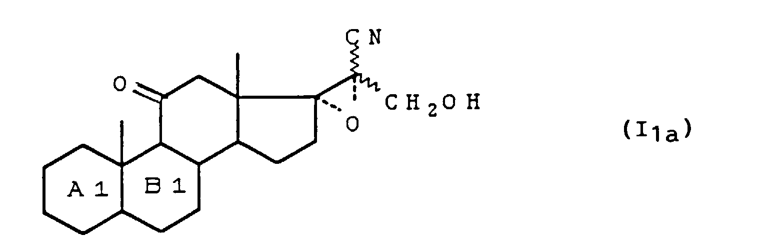 of which the ketone function in position 11 is reduced, if appropriate, in order to obtain a compound of formula (I_{1b}): 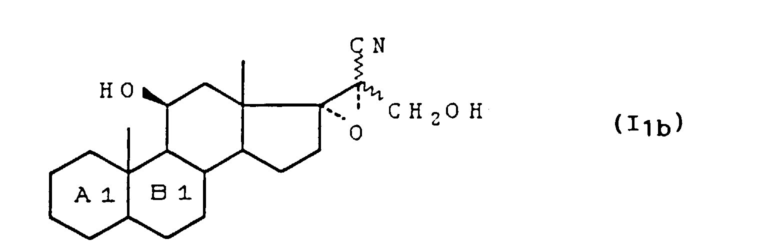 of which compounds of formulae (I₁ₐ) and (I_{1b}) the ketone function in position 3 is released, if appropriate, in order to obtain a corresponding 3-keto compound.

6. Process according to claim 5, characterized in that:
- the ketone function in position 3 is protected in the form of alkylene ketal;
- the epoxidation agent is hydrogen peroxide and the operation is carried out in a basic medium;
- the reducing agent of the ester function and the ketone function is an alkaline borohydride.

7. Use of the compounds of formula (I), as defined in claim 1, for the preparation of compounds of formula (A): 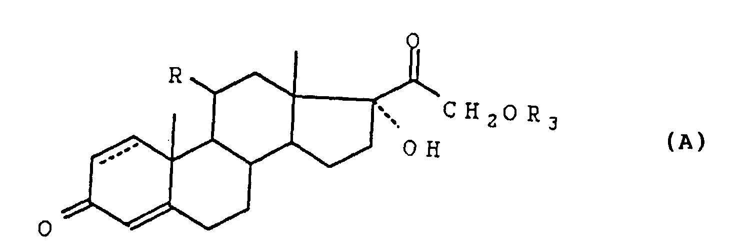 in which R is defined as previously, R₃ represents an alkyl radical containing 1 to 6 carbon atoms and the dotted line represents an optional second carbon-carbon bond, characterized in that the hydroxy function in position 21 of a compound of formula (I) is protected, in order to obtain a compound of formula (VII): 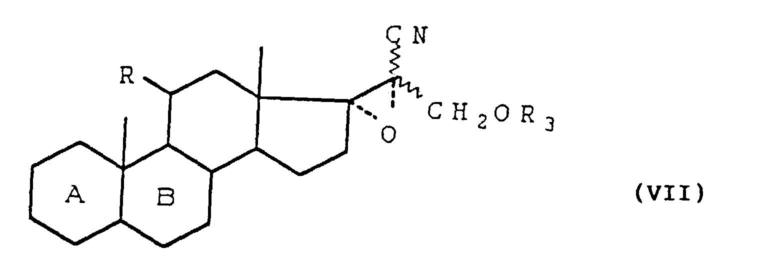 in which R, R₃, A and B have the meaning already indicated, the nitrile function of which is hydrated, in order to obtain a compound of formula (VIII): 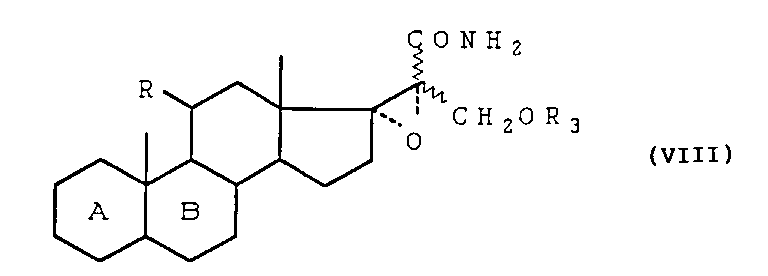 which is subjected to the action of an alkali or alkalineearth hypohalogenite in an aqueous polar organic solvent, in order to obtain a compound of formula (IX): 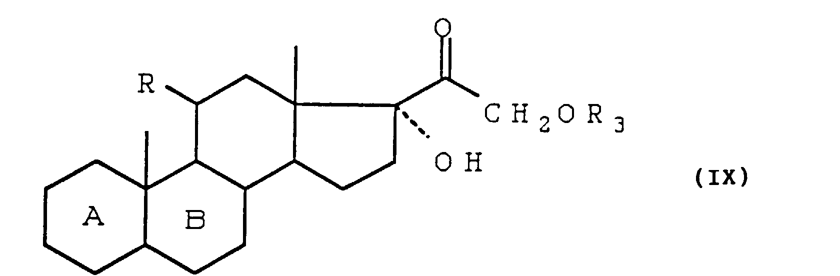 the ketone function in position 3 of which is released, if appropriate, in order to obtain the expected compound of formula (A).

8. Use according to claim 7, characterized in that the degradation is carried out without intermediate isolation of the amide of formula (VIII).

9. Use according to any one of claims 7 and 8, characterized in that the hydration of the nitrile function is carried out by the action of an aqueous mineral base, in a polar solvent.

10. The compounds of formula: 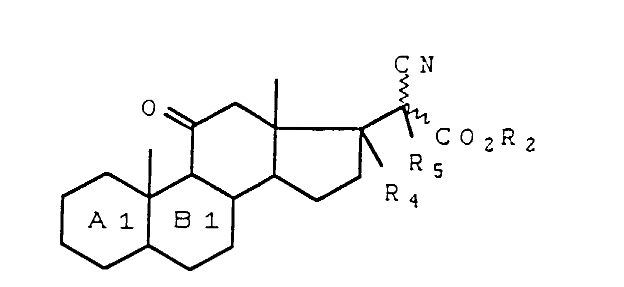 in which A₁, B₁, R₂ and the wavy lines are defined as in claim 5, and R₄ and R₅ form together a carbon-carbon bond or an epoxy in alpha position.

11. The compounds of formula: 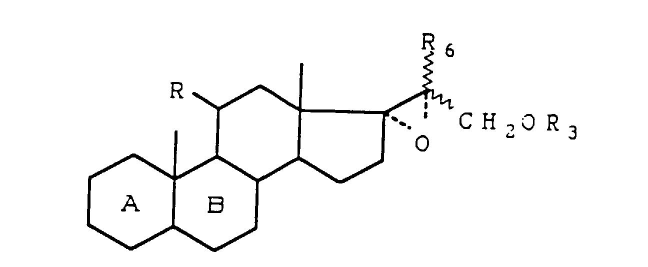 in which A, B, R and the wavy lines are defined as in claim 1, R₃ is defined as in claim 7 and R₆ represents a cyano radical or a carbamoyl radical.
